(19)

**Europäisches Patentamt**
**European Patent Office**
**Office européen des brevets**

(11) **EP 4 405 679 B1**

(12) **EUROPEAN PATENT SPECIFICATION**

(45) Date of publication and mention
of the grant of the patent:
**29.10.2025   Bulletin 2025/44**

(21) Application number: **22790362.2**

(22) Date of filing: **21.09.2022**

(51) International Patent Classification (IPC):
***G01N 33/569*** (2006.01)

(52) Cooperative Patent Classification (CPC):
**G01N 33/56911;** G01N 2333/01; G01N 2333/21;
G01N 2446/00

(86) International application number:
**PCT/IB2022/058928**

(87) International publication number:
**WO 2023/047306 (30.03.2023 Gazette 2023/13)**

(54) **INNOVATIVE BIOTECHNOLOGICAL SYSTEMS FOR THE DETECTION OF CELLULAR OR MOLECULAR ANALYTES**

INNOVATIVE BIOTECHNOLOGISCHE SYSTEME ZUM NACHWEIS ZELLULÄRER ODER MOLEKULARER ANALYTE

SYSTÈMES BIOTECHNOLOGIQUES INNOVANTS POUR LA DÉTECTION D'ANALYTES CELLULAIRES OU MOLÉCULAIRES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **21.09.2021  IT 202100024185**

(43) Date of publication of application:
**31.07.2024   Bulletin 2024/31**

(73) Proprietors:
• **Università Degli Studi Di Messina**
  **98122 Messina (IT)**
• **Alma Mater Studiorum Universita di Bologna**
  **40126 Bologna (IT)**

(72) Inventors:
• **CONOCI, Sabrina**
  **95030, Tremestieri Etneo (CT) (IT)**
• **GUGLIELMINO, Salvatore**
  **95127, Catania (CT) (IT)**
• **CALVARESI, Matteo**
  **40128, Bologna (BO) (IT)**
• **DANIELLI, Alberto**
  **40068, San Lazzaro Di Savena (BO) (IT)**
• **PRODI, Luca**
  **40136, Bologna (BO) (IT)**

• **FAZIO, Enza**
  **98166, Messina (ME) (IT)**
• **PETROSINO, Annapaola**
  **84013, Cava de' Tirreni (SA) (IT)**
• **DI GIOSIA, Matteo**
  **40127, Bologna (BO) (IT)**

(74) Representative: **Robba, Pierpaolo**
**Interpatent S.R.L.**
**Via Caboto, 35**
**10129 Torino (IT)**

(56) References cited:
**EP-A1- 2 474 612     WO-A1-2022/189966**

• **DE PLANO LAURA M. ET AL: "Phage-based
assay for rapid detection of bacterial pathogens
in blood by Raman spectroscopy", JOURNAL OF
IMMUNOLOGICAL METHODS, vol. 465, 2
December 2018 (2018-12-02), NL, pages 45 - 52,
XP055868433, ISSN: 0022-1759, DOI: 10.1016/
j.jim.2018.12.004**

**(Cont. next page)**

- DE PLANO LAURA M ET AL: "Direct conjugation of silicon nanoparticles with M13pVIII-engineered proteins to bacteria identification", APPLIED PHYSICS A, SPRINGER BERLIN HEIDELBERG, BERLIN/HEIDELBERG, vol. 124, no. 11, 30 October 2018 (2018-10-30), XP036966548, ISSN: 0947-8396, [retrieved on 20181030], DOI: 10.1007/S00339-018-2169-1
- MACHERA SEBASTIAN J. ET AL: "Phage-Based Sensors in Medicine: A Review", CHEMOSENSORS, vol. 8, no. 3, 31 July 2020 (2020-07-31), pages 61, XP055912211, DOI: 10.3390/chemosensors8030061
- MOSIER-BOSS P A ET AL: "Use of Fluorescently Labeled Phage in the Detection and Identii cation of Bacterial Species", APPLIED SPECTROSCOPY, vol. 57, no. 9, 1 January 2003 (2003-01-01), XP055912207
- CALABRESE F ET AL: "Phage-coated paramagnetic beads as selective and specific capture system for biosensor applications", 2015 XVIII AISEM ANNUAL CONFERENCE, IEEE, 3 February 2015 (2015-02-03), pages 1 - 4, XP032751430, DOI: 10.1109/AISEM.2015.7066851
- RIZZO M G ET AL: "Rapid detection of bacterial pathogens in blood through engineered phages-beads and integrated Real-Time PCR into MicroChip", SENSORS AND ACTUATORS B: CHEMICAL, ELSEVIER BV, NL, vol. 329, 27 November 2020 (2020-11-27), XP086455479, ISSN: 0925-4005, [retrieved on 20201127], DOI: 10.1016/J.SNB.2020.129227
- ZHENG LEI ET AL: "A New Fusion Peptide Targeting Pancreatic Cancer and Inhibiting Tumor Growth", ONCOTARGETS AND THERAPY, vol. Volume 13, 12 August 2020 (2020-08-12), pages 7865 - 7875, XP093012744, Retrieved from the Internet <URL:https://www.dovepress.com/getfile.php?fileID=60539> DOI: 10.2147/OTT.S246969

**Description**

<u>Technical Field</u>

The present invention relates to a method for detecting cellular or molecular analytes, which method uses two types of engineered phages: a "capture" phage (bait phage) and a "signal" phage (reporter phage). Through this method it is possible to selectively bind a target analyte and thus recognize said target analyte.

<u>Background Art</u>

Within the field of laboratory diagnostic systems in general, for example, clinical and molecular diagnostic systems, there is always a strong interest in developing innovative systems.

The main analytical methodologies currently used for detecting cellular systems (microbes and eukaryotic cells) of analytes of different nature (such as viruses, molecular markers, toxins, proteins, nucleic acids, etc.) are mainly based on indirect detection systems, which, though sensitive (for example, PCR, ELISA, other antibody tests, etc.), provide for manipulations by qualified operators, wait times for getting answers from the test, and centralization of the analyses. De Plano Laura M. et al.: "Phage-based assay for rapid detection of bacterial pathogens in blood by Raman spectroscopy",JOURNAL OF IMMUNOLOGICAL METHODS, vol. 465, 2 December 2018 (2018-12-02), pages 45-52, discloses methods for detecting bacteria using M13 phages as capture agents, while the detection of the bacteria is done via Raman spectroscopy.

Another aspect of interest is the so-called POCT (Point-Of-Care Technologies) technologies, for developing bioengineered systems integrated with innovative biotechnologies. Particularly, such systems allow performing rapid analytical tests in non-laboratory contexts, carried out by non-qualified personnel or even by the patient himself/herself. In particular, the increasing attention to research in the POCT field has been largely driven by two distinct trends in modern society: (i) the general need to reduce high healthcare costs and (ii) the demand for better analytical solutions for early diagnoses and customized therapies. In addition to this, as a result of the burst of the pandemic, the demand for massive screenings, which at the same time should be highly accurate, sensitive and inexpensive, has gained great importance. These aspects are crucial to deal with efficient patient management, timely diagnostics, as well as prompt detection of infectious disease outbreaks, thus making it possible to deal efficiently with pandemic or epidemic events. Therefore, there is still a search going on for analytical systems that are sensitive and selective, and at the same time inexpensive and having direct readout, thus allowing for direct detection of molecular and/or cellular analytes, in particular microorganisms (e.g., bacteria and viruses), in which systems said detection should have high selectivity, specificity and cost-effectiveness.

<u>Summary of Invention</u>

The problem addressed by the present invention is thus to provide a method for detecting analytes such as, for example, cellular systems (for examples, bacteria, parasites, eukaryotic cells), viruses, molecular markers (for example, toxins, proteins, nucleic acids), preferably of bioclinical interest, which method should have characteristics of high sensitivity and specificity, is direct and can also be used in rapid and cost-effective systems.

This problem is solved by the method of the present invention, as outlined in the appended claims, the definitions contained therein being an integral part of the present disclosure.

The Applicant has now found a method for detecting cellular or molecular analytes that uses two types of engineered phages: a bait phage and a reporter phage. Specifically, said phage comprises at least one fusion peptide exposed on the major capsomer (major coat protein) pVIII and capable of selectively binding the target analyte. Subsequently, the reporter phage exposing at least one recognition peptide on the minor capsomer (minor coat protein) pIII, at the phage tip, and at least one marker for signal transduction on the major capsomer, binds in turn the analyte captured by the bait phage. Such detection system based on the two types of engineered phages can be defined as "molecular sandwich".

Therefore, in a first aspect, the present invention relates to a method comprising the steps of:

a) contacting a sample containing the analyte with first phages M13 (bait phages) bound to an appropriate support and comprising at least one peptide or polypeptide or fusion protein specific for recognizing the analyte, said peptide or polypeptide or fusion protein being exposed on the protein pVIII, thus achieving formation of first complexes comprising first phages M13 bound to the analyte ("bait phage-analyte" complexes);

b) separating the first phages M13 from the sample part that, in the preceding step a), did not bind to the first phages M13;

c) adding, to the first phages M13, second phages M13 (reporter phages) comprising at least one peptide or polypeptide or protein for specific recognition of the analyte fused on the protein pIII, and at least one marker conjugated to the capsid of said second phages M13, said marker being selected from a fluorophore, a chromophore, an electrochemically active species or an electrochemiluminescence-active species, thus achieving formation of second complexes comprising first phages M13 and second phages M13 bound to the analyte in a sandwiched manner ("bait phage-analyte-reporter phage" complexes);

d) carrying out a washing to remove the second phages M13 that did not bind the analyte,

e) determining the derived signal generated by the markers of the second phages M13 of said second complexes.

Advantageously, the method according to the invention makes it possible to detect a target analyte in a selective manner, with high sensitivity and in a direct manner. Particularly, said method can be used for direct detection of infectious microorganisms (for example, bacteria, parasites and viruses, for example, SARS-CoV-2). Other advantageous examples include direct detection of human eukaryotic cells, as in the case of variations due to pathologies such as oncological pathologies, as well as analytes of different molecular nature (for example, proteins, molecular markers, toxins, nucleic acids, etc.). Advantageously, the method according to the invention provides for the possibility of detections by multiplexing for the integrated analysis of several analytes at the same time. Also, another advantageous aspect is that this type of detection is inexpensive. Finally, implementation of the method of the invention for POCT is also possible.

Further features and advantages of the method of the invention will be apparent from the description of the exemplary embodiments of the invention, provided as indicative examples of the invention itself.

Brief Description of Drawings

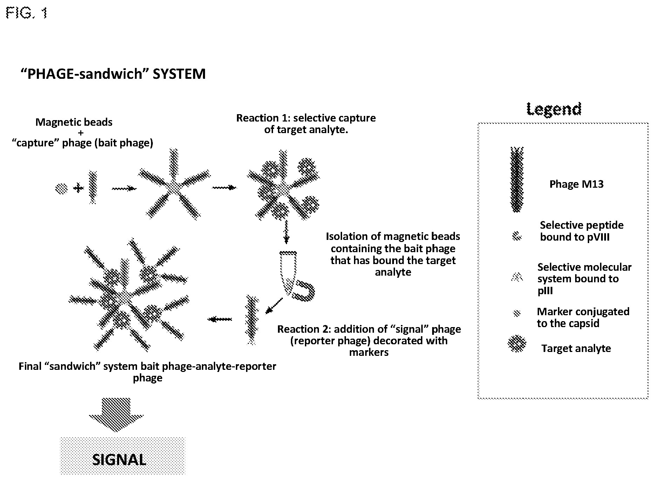

Fig.1  is a schematic illustration of the mechanism of action of the method according to the invention;

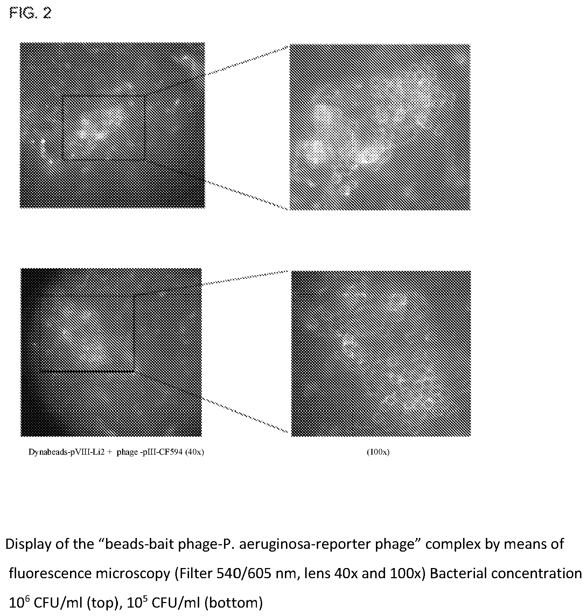

Fig.2  shows an image of the molecular sandwich "bait phage+analyte+reporter phage";

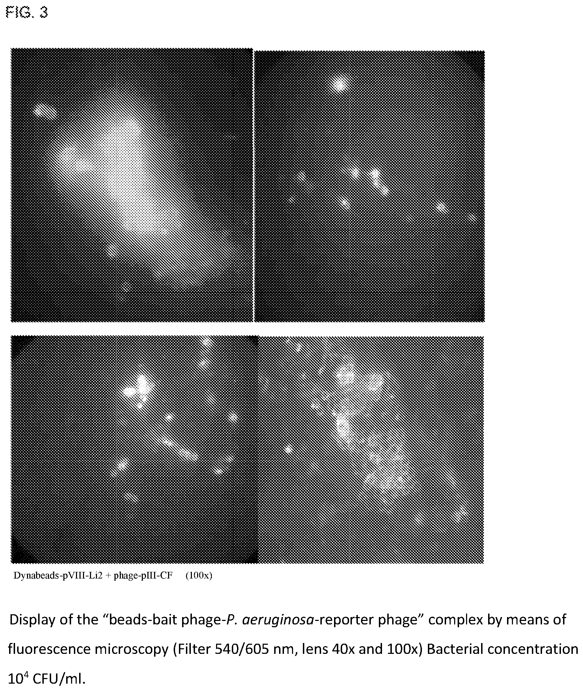

Fig.3  shows a further image of the molecular sandwich "bait phage+analyte+reporter phage";

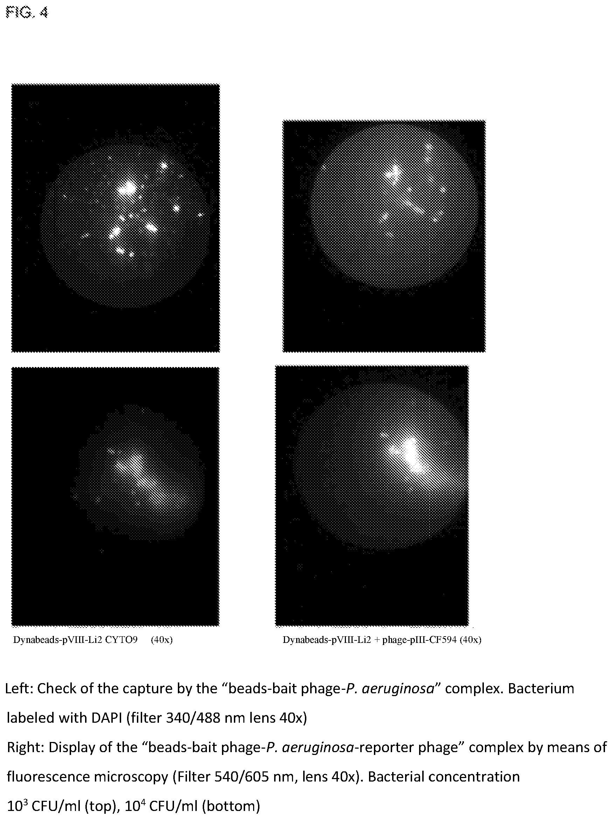

Fig.4  shows a further image of the molecular sandwich "bait phage+analyte+reporter phage";

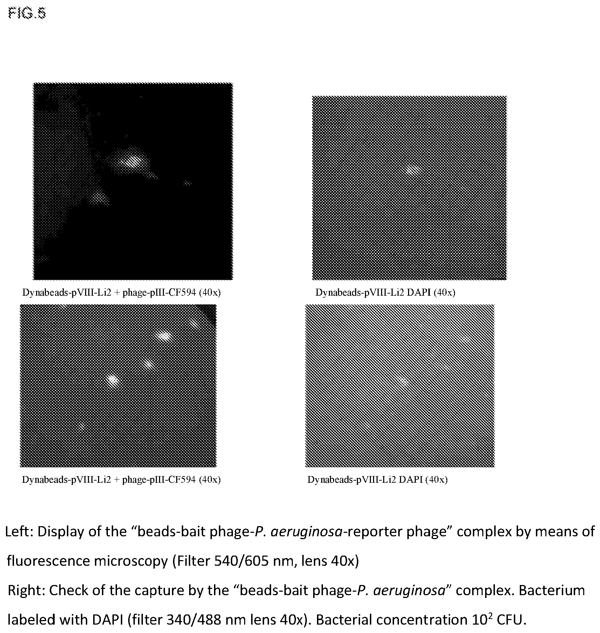

Fig.5  shows a further image of the molecular sandwich "bait phage+analyte+reporter phage";

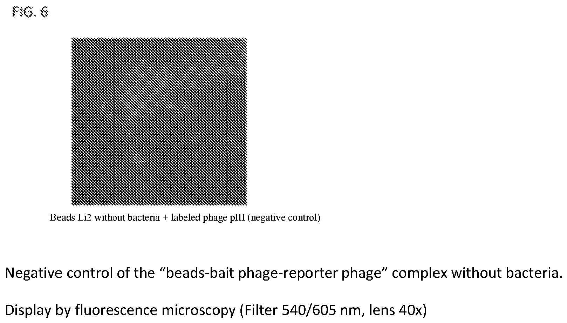

Fig.6  shows a further image of the molecular sandwich "bait phage+analyte+reporter phage".

Description of Embodiments

For the purposes of the invention, the definitions of some terms used in the present description and in the appended claims are provided below.

The term "cellular or molecular analyte" means systems such as, for example, cellular systems (such as microbes, parasites, eukaryotic cells), viruses, molecular markers (such as toxins, proteins, nucleic acids, etc.), preferably of bioclinical interest.

The object of the present invention is a method for detecting an analyte in a sample, wherein said analyte is selected from:

- a cellular system selected from: microbe, parasite, eukaryotic cell, bacterium, virus; or
- a molecular marker selected from: toxin or protein;

said method comprising the steps of:

a) contacting the sample with first phages M13 (bait phages) bound to an appropriate support and comprising at least one peptide or polypeptide or fusion protein specific for recognizing the analyte, said peptide or polypeptide or fusion protein being exposed on the protein pVIII, thus achieving formation of first complexes comprising first phages M13 bound to the analyte ("bait phage-analyte" complexes);

b) separating the first phages M13 from the sample part that did not bind to the first phages M13;

c) adding, to the first phages M13, second phages M13 (reporter phages) comprising at least one peptide or polypeptide or protein for specific recognition of the analyte fused on the protein pIII, and at least one marker conjugated to the capsid of said second phages M13, said marker being selected from a fluorophore, a chromophore, an electrochemically active species or an electrochemiluminescence-active species, thus achieving formation of second complexes comprising first phages M13 and second phages M13 bound to the analyte in a sandwiched manner ("bait phage-analyte-reporter phage" complexes);

d) carrying out a washing to remove the second phages M13 that did not bind the analyte;

e) determining the derived signal generated by the markers of the second phages M13 of said second complexes.

The above-mentioned steps are carried out in the order set forth above.

Preferably, the first phage M13 (bait phage) is an engineered phase M13 exposing at least one fusion peptide, preferably in fusion with the protein "major coat protein pVIII" of the phage, capable of selectively binding the analyte. Preferably, the at least one fusion peptide is exposed in a number of copies in the order of tens or hundreds, up to thousands, of copies on the capsid of the first phage M13.

The second phage M13 (reporter phage), is a phage M143 engineered with methods known to the person skilled in the art and exposing at least one peptide or polypeptide or fusion protein, preferably in fusion with the "minor coat protein pIII" of the phage, capable of selectively binding the analyte. The protein "minor coat protein pIII" is a protein of the capsid of said second phage M13.

With reference to the marker, it allows determining the derived signal generated by the marker. Such signal determination can take place by optical or electronic detection of the analyte. In particular, the at least one marker can be detected by means of photometry, fluorimetry, photoluminescence, thermoluminescence, chemiluminescence, electrochemiluminescence, voltammetry and other electrochemical techniques. In fact, after the passage at step c), the signal of effected "capture" of the analyte can be detected by means of optical or electronic sensors, depending on the marker used. According to a preferred aspect, the marker conjugated to the capsid of the second phage M13 is a fluorophore, a chromophore, an electrochemically active species or an electrochemiluminescence-active species. According to a preferred aspect of the invention, the marker is a fluorophore, the fluorophore preferably being CF594 (Biotium).

According to a preferred aspect of the invention, the analyte is a bacterium, preferably a Gram-negative bacterium, even more preferably *P. aeruginosa.* Preferably, the at least one peptide or polypeptide or fusion protein of step a) has the sequence SEQ. ID NO: 1 (QRKLAAKLT). Preferably, the at least one peptide or polypeptide or phase recognition protein of step c) has the sequence SEQ. ID NO: 2 (KLAKLAKKLAKLAK).

According to a preferred aspect of the invention, when the analyte is *P. aeruginosa,* the at least one peptide or polypeptide or fusion protein of step a) has the sequence SEQ. ID NO: 1 (QRKLAAKLT). Preferably, said at least one fusion peptide is exposed in a number of copies in the order of tens or hundreds, up to thousands, of copies on the capsid of the first phage M13.

According to a preferred aspect of the invention, when the analyte is *P. aeruginosa,* the at least one peptide or polypeptide or phage recognition protein of step c) has the sequence SEQ. ID NO: 2 (KLAKLAKKLAKLAK).

According to a preferred aspect of the invention, when the analyte is *P. aeruginosa,* the at least one peptide or polypeptide or fusion protein of step a) has the sequence SEQ. ID NO: 1 (QRKLAAKLT) and the at least one peptide or polypeptide or phage recognition protein of step c) has the sequence SEQ. ID NO: 2 (KLAKLAKKLAKLAK).

According to a preferred aspect, a linker can be used. Advantageously, the linker confers characteristics of mobility to the peptide. Preferably, the linker is GGGS.

In a preferred aspect of the method according to the invention, a support, for example, a magnetic microsphere (MNP), is used, said support being functionalized with a bait phage (thus obtaining an MNP-bait phage conjugate), wherein said phage comprises at least one fusion peptide exposed on the major capsomer (major coat protein) pVIII and capable of selectively binding the target analyte. Also, exemplary but not limiting procedures of functionalization are set forth below. Therefore, in the detection method according to the invention, a support that is functionalized with the first phage M13 can be used. Preferably, the support is a magnetic microsphere (MNP). Herein, the term "magnetic microspheres", commonly referred to in the art as magnetic microparticles MNP, means particles on which one or more phages can be immobilized. As a person skilled in the art will understand, herein said particles can also be referred to by interchangeably using the term sphere/spheres and corresponding terms (for example, microspheres). Such particles can be inorganic particles, preferably of ferrite or the like, and can be functionalized with one or more phages. As a result of said functionalization, a biosensor can be obtained. In particular, the microspheres are advantageous because of their tridimensional structure with a ratio between surface area and volume higher than that of a plan surface, thus providing more binding sites for the target. Another advantage is their easiness of handling in fluids, as well as their cost-effectiveness. According to a preferred aspect, the selected phages exposing the active recognition peptides fused to the protein pVIII are covalently bound on the magnetic microspheres. This is of advantage for use in the capture, washing and rapid concentration of cells, viruses or proteins directly in the sample under test.

According to a preferred aspect, if the support used for the first phages M13 consists of magnetic microspheres, the step of separation of the first phages M13 from the sample part not bound to the first phages M13 takes place by means of magnetic capture of the magnetic microspheres.

According to a further preferred aspect, the support for the first phages M13 is an electrode, a polymer or a silicon-based surface. In this case, the step of separation of the first phages M13 from the sample part not bound to the first phages M13 takes place by washing.

Preferred applications of the method of the invention are aimed at direct detection of cells of microorganisms with special reference to infectious microorganisms (bacteria and viruses), such as pathogenic Gram-negative bacteria (e.g., species listed in the WHO's "priority list") or viruses such as SARS-CoV-2. Such method can also be extended to eukaryotic cells, especially in liquid biopsy with the possibility of capturing, concentrating and directly detecting cancer cells. In this case, the selectivity of the method will be related to the specificity of the engineered bait phages and reporter phages towards specific tumor markers exposed on the surface of the cells. Such method can also be extended to molecular analytes, such as toxins, proteins and nucleic acids, especially when these can be considered as biomarkers for certain pathologies. The development of such "phage probes" and, accordingly, the flexibility of the analytical platform presented, is favored by the large adaptability of the phage-display schemes allowing to select, by biopanning, peptide and antibody libraries (scFvs, single-domain antibodies, etc.) following immobilization of the proteins of interest (either native or recombinant) on the aforementioned supports.

The above is to be understood as being exemplary and not limiting. In addition, the person skilled in the art will be able to understand that modifications can be made without departing from the scope of the present invention, which is defined by the appended claims.

Examples

*Reagents used and preparation thereof*

[Table 1]

| Buffer A: borate buffer 0.1 M, pH 9.5<br>6.18 g H3BO3 (MW 61.83). Dissolve in 800 mL distilled water. Adjust the pH to 9.5 by using 5M NaOH and adjust the volume to 1 L with distilled water. |
| --- |
| Buffer B: 0.1 M Na-phosphate buffer, pH 7.4<br>2.62 g NaH2PO4. H2O (MW 137.99) and 14.42 g Na2HPO4. 2 H2O (MW 177.99). Adjust the volume to 1 L with distilled water. |
| Buffer C: 3 M ammonium sulphate in buffer A or B<br>39.64 g (NH4)2SO4 dissolved in buffer A or B. Adjust the pH with NaOH or HCl. Adjust to 100 mL with buffer A or B. |
| Buffer D<br>PBS pH 7.4 with 0.5% (w/v) BSA<br>Add 0.88 g NaCl (MW 58.4) and 0.5% (w/v) BSA to 80 mL 0.01 M sodium phosphate, pH 7.4. Mix thoroughly and adjust the volume to 100 mL with 0.01 M sodium phosphate pH 7.4. |
| Buffer E<br>PBS pH 7.4 with 0.1% (w/v) BSA<br>Add 0.88 g NaCl (MW 58,4) and 0.1% (w/v) BSA to 80 mL of 0.01 M sodium phosphate<br>pH 7,4. Mix thoroughly and adjust the volume to 100 mL with 0.01 M sodium phosphate, pH 7.4. |

Experimental procedure for functionalization

50 $\mu$l Dynabeads M-280 Tosylactivated (Invitrogen cat. 142.03) were placed in roundbottomed Eppendorf tubes and washed twice with 500 $\mu$l borate buffer (0.1 M borate buffer pH 9.5), for 5 minutes under gentle stirring on a wheel. The beads were collected on a magnetic device for 10 minutes, then the buffer was discarded and the beads were resuspended in 50 $\mu$l borate buffer. 30 $\mu$l of the bait phage from stock $1 \times 10^{12}$ TU/ml, i.e. $3 \times 10^{6}$ phage clones, in borate buffer were added and then 60 $\mu$l borate buffer and 60 $\mu$l ammonium sulphate buffer (3M pH 7.4) were added. The tube was incubated at 37°C for 24 h under gentle stirring in a rotating tumbler mixer with 30° inclination. The beads were separated on the magnetic device for 10 minutes, the supernatant was discarded and the beads were washed twice with 500 $\mu$l PBS buffer pH 7.4 + 1% BSA for 5 minutes under stirring on a wheel. Then 500 $\mu$l blocking buffer (PBS pH 7.4 + 4% BSA) were added and the beads were stirred on a wheel for 2 h at room temperature (RT). The beads were separated on the magnetic device for 10 minutes, the supernatant was discarded and the beads were washed twice with 500 $\mu$l PBS buffer pH 7.4 + 1% BSA for 5 minutes in a rotating tumbler mixer. Finally, the beads were resuspended in 200 $\mu$l PBS and left to stand at 4°C (Dynabeads-bait phage). Three different stocks were prepared.

*ELISA test for verifying the functionalization of Dynabeads with the bait phage*

In order to verify proper functionalization of the beads with the bait phage, an ELISA test was carried out by using an anti-phage M13 antibody. In brief, 20 $\mu$l functionalized beads were washed twice with 500 $\mu$l washing buffer (PBS pH 7,4 + 0.5 % Tween 20) for 5 minutes on a wheel. The beads were resuspended in 100 $\mu$l PBS and 70 $\mu$l anti-M13 HPR antibody (1:2500 in PBS + 0.1% BSA + 0.5% Tween 20) were added, and then the beads were incubated in a rotating tumbler mixer at 37°C for 1 h. Then 5 washings were carried out in 500 $\mu$l di PBS 0.5% Tween. In the last step, the beads were resuspended in 250 $\mu$l TMB and incubated for 20 minutes on a wheel at room temperature (RT) in the dark. After complete development, the reaction was quenched with 31 $\mu$l 6N H2SO4. The beads were placed on the magnet for 10 minutes and then 200 $\mu$l of the *in-toto* solution and of a 1:10 dilution were read at 450 nm.

Results obtained:

[Table 2]

|  | Reading values at 450 nm | |
|---|---|---|
|  | Undiluted solution (*toto*) | Diluted 1:10 |
| Dynabeads-bait phage | 2.415 | 1.407 |

*Capture test for P. aeruginosa with phage system Dynabeads/phage-pVIII*

*Capture tests*

For capture and detections tests, an ON culture in 5 ml of LB of isolation *P. aeruginosa* ATCC 27853 on a cetrimide agar plate was used. The bacteria were collected by centrifugation at 8000 xg for 10 minutes and resuspended in an isovolume of PBS. The bacteria were diluted in PBS to obtain a bacterial stock with OD600 of 0.29 (equal to $10^8$ cells/ml). 20 $\mu$l Dynabeads-bait phage were washed twice on a wheel for 5 minutes with 500 $\mu$l PBS, then they were placed on the magnet for 10 minutes, the supernatant was discarded and they were resuspended in 1 ml PBS and then 100 $\mu$l of the bacterial suspension $10^8$ cells/ml (final concentration $10^7$ cells) or 100 $\mu$l PBS (negative control) were added.
The sample was placed in an inclined wheel at 37°C for 30 minutes.
The beads-bait phage with any captured bacteria were separated on the magnetic device for 10 minutes and then the supernatant was taken out and the beads-phage-bacteria complex was resuspended in 50 $\mu$l PBS and used for recognition with phage-pIII-CF594. The uncaptured bacteria present in the supernatant were titred by means of an on-plate dilution method and count of CFUs on Cetrimide agar to evaluate the bacterial capture efficiency on the Dynabeads functionalized with the bait phage. All tests were carried out in triplicate. The efficiency capture of the cells of *P. aeruginosa* with the Dynabeads-bait phage in the carried out tests (calculated as the percentage of input number of bacteria - number of bacteria in the supernatant = number of captured bacteria) was on average 90%.

*Engineering of the reporter phage (phage-pIII-CF594)*

*Construction of the reporter phage of Gram-negative bacteria*

The sequence coding for the peptide KLAKLAKKLAKLAK, capable of binding the outer membrane of Gram(-) bacteria was cloned in frame to a short linker GGGS upstream of the gene p3 of the phagemid pSEX81 (ProGen) in order to allow pentavalent display thereof on the tip of the detecting phage-pIII. An exponential phase culture of E. coli TG1 transformed with the aforesaid recombinant phagemid was then superinfected with the helper phage Hyperphage (ProGen) to allow production of the phage-pIII virions redirected to the recognition of Gram(-) bacteria. Following purification by precipitation in NaCl/PEG solution, the phages-pIII were resuspended in PBS pH 7.4 at a target concentration of about $10^{13}$ phages/ml. Said redirected phages-pIII were conjugated to the fluorophore CF594.

*Conjugation of the reporter phage to the fluorophore CF594*

The method of conjugating an imaging agent, being CF594, to the capsid of the detecting phage-pIII is described below. A stock solution of 10 mM CF594 succinimide ester in DMSO at room temperature is prepared.
10 $\mu$l of the stock solution are added to 1 ml of solution containing the reporter phage-pIII in 0.1 M bicarbonate buffer at pH 8.3, under constant stirring with a magnetic stirrer. The succinimidyl ester group of the dye reacts with the amine groups of the capsid proteins of the phage to form a stable amide bond. The mixture is kept under stirring at room temperature overnight.

The functionalized phage is separated from the unconjugated fraction of CF594 by dialysis (cut-off 14 kDa) in PBS buffer. 3 cycles of dialysis of 8 h each were carried out using 1 ml of solution of bioconjugate to be dialyzed and 500 ml PBS.

The quantification of phages was carried out by reading the UV-Vis absorbance spectrum of the phages, obtaining values for the wavelengths of 269 nm e 320 nm and then applying the following formula:

$$\text{Phage concentration (virions/mL)} = (A269-A320)*6*10^{16}/ \text{ bp phage genome}=2.04E+13$$

In order to quantify the CF594 conjugated to the phage, the following formula has been applied:

conc. CF594 (microM) = A594/epsilon594 = 8.91
Given the concentration of CF594 and the concentration of phages (virions/ml), the ratio CF594 molecules/phage is obtained, corresponding to 264.

Verification of the redirection of the reporter phage-pIII-CF594 by cytofluorimetry

The redirection of the phages-pIII-CF594 to *P. aeruginosa* (and other Gram-negative bacteria) was verified by performing cytofluorimetry assays using a BioRad S3e Cell Sorter: $10^{10}$ virions of the phage-pIII-CF594 were incubated for 20-30 minutes with $10^9$ cells of *P. aeruginosa* PA14 resuspended in 1 ml PBS pH 7.4. The non-redirected phage M13K07 (New England Biolabs) conjugated to CF594 was used as negative control for the cytofluorimetric analyses. The MFI (mean fluorescence intensity) detected for the phage-pIII-CF594 resulted increased by over 4 times with respect to the control M13K07-CF594, thus validating the fact that the detecting phage had been redirected to PA14. The cytofluorimetric analysis also made it possible to ascertain that one third of the population of *P. aeruginosa* had a fluorescence intensity increased by ~ one order of magnitude (1 Log) following addition of the reporter phage-pIII-CF594. This is an index of the capability of the detecting phage to mediate the accumulation of several fluorophores near the Gram(-) bacterial cell.

*Capture and detection tests with double phage system (Dynabeads/bait phage-pVIII + reporter phage-pIII-CF594)*

*Labeling with reporter phage-pIII-CF594*

To the complex beads-phage-bacteria resuspended in 50 µl PBS and to the negative control sample (without bacteria), 2 µl phage-pIII-CF594 (labeled stock 1:1, 2.04E+13 phages/ml, 0.91 µM CF594, 246 fluo/phage) were added and placed in a rotating mixer for 40 minutes at 37°C, in the last 5 minutes of reaction 1 µl DAPI (stock 10 mg/ml) or SYTO9 were added to label the captured bacterial cells. Each sample was placed on a magnet for 10 minutes, the supernatant was discarded, gentle washing was carried out in 100 µl PBS at RT for 5 minutes without stirring. The sample were then placed again on the magnet for 10 minutes, the supernatant was discarded, and gentle resuspension was carried out in 50 µl PBS. 10 µl of each sample were placed on a slide, and the samples were displayed with an Olympus fluorescence microscope (filter 540/605 lens 40x and 100x) and the images were captured by means of a CCD camera.

*Comments on the results*

The results obtained with the labeling of the captured bacteria with the reporter phage pIII CF 594 indicate a specific recognition of the phage pIII on *P. aeruginosa* complexed on the Dynabeads functionalized with the bait phage.

**Claims**

1.  Method for detecting an analyte in a sample, wherein said analyte is selected from:

    - a cellular system selected from: microbe, parasite, eukaryotic cell, bacterium, virus; or
    - a molecular marker selected from: toxin, protein or nucleic acids;

    said method comprising the steps of:

    a) contacting the sample with first phages M13 (bait phages) bound to an appropriate support and comprising at least one peptide or polypeptide or fusion protein specific for recognizing the analyte, said peptide or polypeptide or fusion protein being exposed on the protein pVIII, thus achieving formation of first complexes comprising first phages M13 bound to the analyte ("bait phage-analyte" complexes);

b) separating the first phages M13 from the sample part that did not bind to the first phages M13;

c) adding, to the first phages M13, second phages M13 (reporter phages) comprising at least one peptide or polypeptide or protein for specific recognition of the analyte fused on the protein pIII, and at least one marker conjugated to the capsid of said second phages M13, said marker being selected from a fluorophore, a chromophore, an electrochemically active species or an electrochemiluminescence-active species, thus achieving formation of second complexes comprising first phages M13 and second phages M13 bound to the analyte in a sandwiched manner;

d) carrying out a washing to remove the second phages M13 that did not bind the analyte,

e) determining the derived signal generated by the markers of the second phages M13 of said second complexes.

2. Method according to claim 1, wherein the analyte is a bacterium.

3. Method according to claim 2, wherein the analyte is a Gram-negative bacterium.

4. Method according to claim 3, wherein the analyte is the Gram-negative bacterium *P. aeruginosa.*

5. Method according to claim 4, wherein the at least one peptide or polypeptide or fusion protein of step a) has the sequence SEQ. ID NO: 1 (QRKLAAKLT).

6. Method according to claim 4, wherein the at least one peptide or polypeptide or phage recognition protein of step c) has the sequence SEQ. ID NO: 2 (KLAKLAKKLAKLAK).

7. Method according to any of the preceding claims, wherein the marker is a fluorophore.

8. Method according to claim 7, wherein the fluorophore is CF594 (Biotium).

9. Method according to any of the preceding claims, wherein the support comprises magnetic microspheres (MNP), said magnetic microspheres being functionalized with said first phages M13.

10. Method according to claim 9, wherein said step of separating the first phages M13 from the sample part that did not bind to the first phages M13 takes place by magnetic capture of the magnetic microspheres.

11. Method according to claim 1, wherein the support is an electrode, a polymer or a silicon-based surface.

12. Method according to claim 11, wherein said step of separating the first phages M13 from the sample part that did not bind to the first phages M13 takes place by washing.

**Patentansprüche**

1. Verfahren zum Nachweis eines Analyten in einer Probe, wobei der Analyt ausgewählt ist aus:

- einem zellulären System, das ausgewählt ist aus Mikrobe, Parasit, eukaryotische Zelle, Bakterium, Virus, oder
- einem molekularen Marker, der ausgewählt ist aus Toxin, Protein oder Nukleinsäure,

wobei das Verfahren die folgenden Schritte aufweist:

a) Kontaktieren der Probe mit ersten Phagen M13 (Köderphagen), die an einen geeigneten Träger gebunden sind und wenigstens ein für den Nachweis des Analyten spezifisches Peptid oder Polypeptid oder Fusionsprotein enthalten, wobei das Peptid oder Polypeptid oder Fusionsprotein auf dem Protein pVIII zugänglich ist, so dass die Bildung von ersten Komplexen erzieht wird, die erste an den Analyten gebundene Phagen M13 ("Köderphagen-Analyt"-Komplexe) enthalten,

b) Trennen der ersten Phagen M13 von dem Teil der Probe, der sich nicht mit den ersten Phagen M13 verbunden hat,

c) Hinzufügen von zweiten Phagen M13 (Meldephagen), die wenigstens ein Peptid oder Polypeptid oder Protein für die spezifische Erkennung des mit dem Protein pIII verbundenen Analyten enthalten, und wenigstens einen Marker, der zu dem Kapsid der zweiten Phagen M13 konjugiert ist, zu den ersten Phagen M13, wobei der Marker ausgewählt ist aus einer fluorophoren, einer chromophoren, einer elektrochemisch aktiven Sorte oder einer elektrochemilumineszenz-aktiven Sorte, so dass die Bildung des zweiten Komplexes erreicht wird, der erste Phagen M13 und zweite Phagen M13 enthält, die sandwichartig an den Analyten gebunden sind,

d) Durchführen einer Waschung, um die zweiten Phagen M13, die sich nicht mit dem Analyten verbunden haben, zu entfernen,

e) Bestimmen des abgeleiteten Signals, das von den Markern der zweiten Phagen M13 des zweiten Komplexes erzeugt wurde.

2. Verfahren nach Anspruch 1, wobei der Analyt ein Bakterium ist.

3. Verfahren nach Anspruch 2, wobei der Analyt ein gramnegatives Bakterium ist.

4. Verfahren nach Anspruch 3, wobei der Analyt das gramnegative Bakterium *P. aeruginosa* ist.

5. Verfahren nach Anspruch 4, wobei das wenigstens eine Peptid oder Polypeptid oder Fusionsprotein von Schritt a) die Sequenz SEQ. ID NO: 1 (QRKLAAKLT) hat.

6. Verfahren nach Anspruch 4, wobei das wenigstens eine Peptid oder Polypeptid oder Phagenerkennungsprotein von Schritt c) die Sequenz SEQ. ID NO: 2 (KLAKLAKKLAKLAK) hat.

7. Verfahren nach einem der vorstehenden Ansprüche, wobei der Marker ein Fluorophor ist.

8. Verfahren nach Anspruch 7, wobei der Fluorophor CF594 (Biotium) ist.

9. Verfahren nach einem der vorstehenden Ansprüche, wobei der Träger magnetische Mikrosphären (MNP) enthält, die mit den ersten Phagen M13 funktionalisiert sind.

10. Verfahren nach Anspruch 9, wobei der Schritt des Separierens der ersten Phagen M13 von dem Teil der Probe, der sich nicht den ersten Phagen M13 verbunden hat, durch magnetische Erfassung der magnetischen Mikrosphären erfolgt.

11. Verfahren nach Anspruch 1, wobei der Träger eine Elektrode, ein Polymer oder eine Oberfläche auf Silikonbasis ist.

12. Verfahren nach Anspruch 11, wobei der Schritt des Separierens der ersten Phagen M13 von dem Teil der Probe, der sich nicht mit den ersten Phagen M13 verbunden hat, durch Waschen erfolgt.

**Revendications**

1. - Procédé de détection d'un analyte dans un échantillon, dans lequel ledit analyte est choisi parmi :

   - un système cellulaire choisi parmi : microbe, parasite, cellule eucaryote, bactérie, virus ; ou
   - un marqueur moléculaire choisi parmi : toxine, protéine ou acides nucléiques ;

   ledit procédé comprenant les étapes consistant à :

   a) mettre en contact l'échantillon avec des premiers phages M13 (phages appâts) liés à un support approprié et comprenant au moins un peptide ou polypeptide ou protéine de fusion spécifique pour reconnaître l'analyte, ledit peptide ou polypeptide ou protéine de fusion étant exposé à la protéine pVIII, permettant ainsi la formation de premiers complexes comprenant les premiers phages M13 liés à l'analyte (complexes "phage appât-analyte") ;
   b) séparer les premiers phages M13 de la partie d'échantillon qui ne s'est pas liée aux premiers phages Ml3 ;
   c) ajouter, aux premiers phages M13, des seconds phages M13 (phages rapporteurs) comprenant au moins un peptide ou polypeptide ou protéine pour une reconnaissance spécifique de l'analyte fusionné à la protéine pIII, et au moins un marqueur conjugué à la capside desdits seconds phages M13, ledit marqueur étant choisi parmi un fluorophore, un chromophore, une espèce électrochimiquement active ou une espèce active par électrochimiluminescence, permettant ainsi la formation de seconds complexes comprenant les premiers phages M13 et les seconds phages M13 liés à l'analyte d'une manière en sandwich ;

d) efffectuer un lavage pour retirer les seconds phages M13 qui ne se sont pas liés à l'analyte ;

e) déterminer le signal dérivé généré par les marqueurs des seconds phages M13 desdits seconds complexes.

2. - Procédé selon la revendication 1, dans lequel l'analyte est une bactérie.

3. - Procédé selon la revendication 2, dans lequel l'analyte est une bactérie à Gram négatif.

4. - Procédé selon la revendication 3, dans lequel l'analyte est la bactérie à Gram-négatif *P.aeruginosa.*

5. - Procédé selon la revendication 4, dans lequel ledit au moins un peptide ou polypeptide ou protéine de fusion de l'étape a) a la séquence SEQ. ID NO : 1 (QRKLAAKLT).

6. - Procédé selon la revendication 4, dans lequel ledit au moins un peptide ou polypeptide ou protéine de reconnaissance de phage de l'étape c) a la séquence SEQ. ID NO : 2 (KLAKLAKKLAKLAK).

7. - Procédé selon l'une quelconque des revendications précédentes, dans lequel le marqueur est un fluorophore.

8. - Procédé selon la revendication 7, dans lequel le fluorophore est le CF594 (Biotium).

9. - Procédé selon l'une quelconque des revendications précédentes, dans lequel le support comprend des microsphères magnétiques (MNP), lesdites microsphères magnétiques étant fonctionnalisées par lesdits premiers phages Ml3.

10. - Procédé selon la revendication 9, dans lequel l'étape de séparation des premiers phages M13 à partir de la partie d'échantillon qui ne s'est pas liée aux premiers phages M13 a lieu par capture magnétique des microsphères magnétiques.

11. - Procédé selon la revendication 1, dans lequel le support est une électrode, un polymère ou une surface à base de silicium.

12. - Procédé selon la revendication 11, dans lequel ladite étape de séparation des premiers phages M13 à partir de la partie d'échantillon qui ne s'est pas liée aux premiers phages Ml3 a lieu par lavage.

FIG. 1

**"PHAGE-sandwich" SYSTEM**

Magnetic beads
+
"capture" phage (bait phage)

Reaction 1: selective capture
of target analyte.

Isolation of magnetic beads
containing the bait phage
that has bound the target
analyte

Reaction 2: addition of "signal" phage
(reporter phage) decorated with
markers

Final "sandwich" system bait phage-analyte-reporter
phage

SIGNAL

**Legend**

Phage M13

Selective peptide
bound to pVIII

Selective molecular
system bound to
pIII

Marker conjugated
to the capsid

Target analyte

FIG. 2

Dynabeads-pVIII-Li2 + phage -pIII-CF594 (40x)                    (100x)

Display of the "beads-bait phage-P. aeruginosa-reporter phage" complex by means of fluorescence microscopy (Filter 540/605 nm, lens 40x and 100x) Bacterial concentration $10^6$ CFU/ml (top), $10^5$ CFU/ml (bottom)

FIG. 3

Dynabeads-pVIII-Li2 + phage-pIII-CF    (100x)

Display of the "beads-bait phage-*P. aeruginosa*-reporter phage" complex by means of fluorescence microscopy (Filter 540/605 nm, lens 40x and 100x) Bacterial concentration $10^4$ CFU/ml.

FIG. 4

Dynabeads-pVIII-Li2 CYTO9    (40x)                    Dynabeads-pVIII-Li2 + phage-pIII-CF594 (40x)

Left: Check of the capture by the "beads-bait phage-*P. aeruginosa*" complex. Bacterium labeled with DAPI (filter 340/488 nm lens 40x)

Right: Display of the "beads-bait phage-*P. aeruginosa*-reporter phage" complex by means of fluorescence microscopy (Filter 540/605 nm, lens 40x). Bacterial concentration $10^3$ CFU/ml (top), $10^4$ CFU/ml (bottom)

FIG.6

Dynabeads-pVIII-Li2 + phage-pIII-CF594 (40x)

Dynabeads-pVIII-Li2 DAPI (40x)

Dynabeads-pVIII-Li2 + phage-pIII-CF594 (40x)

Dynabeads-pVIII-Li2 DAPI (40x)

Left: Display of the "beads-bait phage-*P. aeruginosa*-reporter phage" complex by means of fluorescence microscopy (Filter 540/605 nm, lens 40x)

Right: Check of the capture by the "beads-bait phage-*P. aeruginosa*" complex. Bacterium labeled with DAPI (filter 340/488 nm lens 40x). Bacterial concentration $10^2$ CFU.

FIG. 6

Beads Li2 without bacteria + labeled phage pIII (negative control)

Negative control of the "beads-bait phage-reporter phage" complex without bacteria.

Display by fluorescence microscopy (Filter 540/605 nm, lens 40x)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **DE PLANO LAURA M. et al.** Phage-based assay for rapid detection of bacterial pathogens in blood by Raman spectroscopy. *JOURNAL OF IMMUNOLOGICAL METHODS*, 02 December 2018, vol. 465, 45-52